# FASCICULE DE BREVET EUROPEEN

(11) **EP 1 589 005 B1**
(45) Date de publication et mention de la délivrance du brevet: **28.03.2007**
(21) Numéro de dépôt: 05290381.2
(22) Date de dépôt: 21.02.2005
(51) Int. Cl.: C07D 223/16

(54) **Procédé de synthèse de l'ivabradine et de ses sels d'addition à un acide pharmaceutiquement acceptable**
Verfahren zur Herstellung von Ivabradine und deren pharmazeutisch verträgliche Säureadditionssalze
Process for the synthesis of Ivabradine and its pharmaceutically acceptable acid addition salts

(30) Priorité: 13.04.2004 FR 0403830
(43) Date de publication de la demande: 26.10.2005
(73) Titulaire: Les Laboratoires Servier, 92415 Courbevoie Cédex (FR)
(72) Inventeur: Lerestif, Jean-Michel, 76190 Yvetot (FR); Lecouve, Jean-Pierre, 76600 Le Havre (FR); Souvie, Jean-Claude, 76600 Le Havre (FR); Brigot, Daniel, 76190 Sainte-Marie-des-Champs (FR); Horvath, Stéphane, 45380 La-Chapelle-Saint-Mesmin (FR); Auguste, Marie-Noelle, 45000 Orleans (FR); Damien, Gérard, 45130 Meung-sur-Loire (FR)

(56) Documents cités:
- EP-A- 0 534 859

## Description

La présente invention concerne un procédé de synthèse industrielle de l'ivabradine de formule (I) : 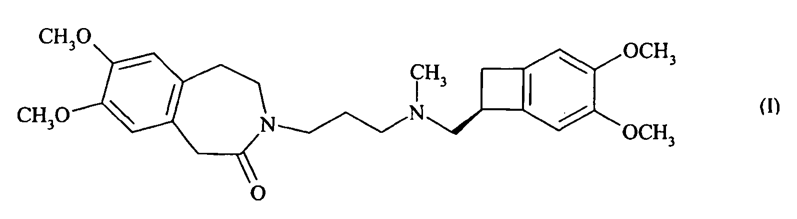 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de ses hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Ce brevet décrit la synthèse du chlorhydrate de l'ivabradine par réaction du composé de formule (II) : 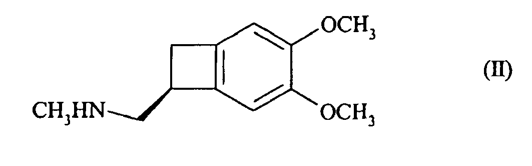 avec le composé de formule (III) : 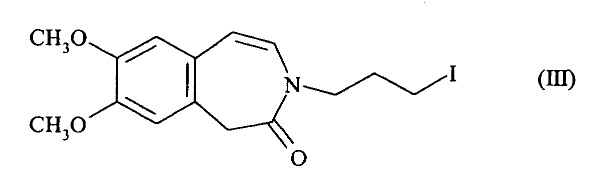 pour conduire au composé de formule (IV) : 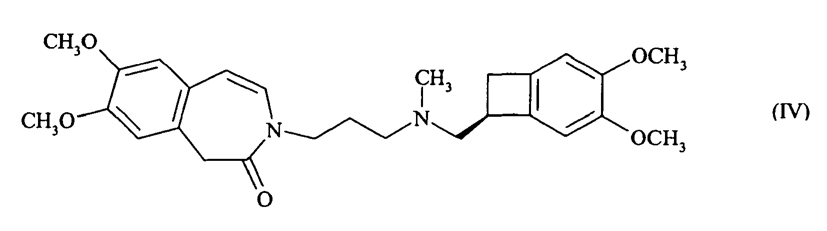 dont l'hydrogénation catalytique conduit à l'ivabradine, qui est alors transformée en son chlorhydrate.

Cette méthode a l'inconvénient de ne conduire au chlorhydrate de l'ivabradine qu'avec un rendement très faible, inférieur à 17 % sur l'ensemble des 3 étapes.

Compte-tenu de l'intérêt pharmaceutique de l'ivabradine et de ses sels, et plus particulièrement de son chlorhydrate, il était important de pouvoir y accéder avec un procédé de synthèse industrielle performant, comportant un nombre minimal d'étapes, et permettant l'obtention de l'ivabradine et de ses sels, et plus particulièrement son chlorhydrate, avec un rendement satisfaisant.

La Demanderesse a présentement mis au point un procédé de synthèse industrielle, permettant l'obtention des sels de l'ivabradine en une seule étape à partir d'un sel du composé de formule (II), avec un très bon rendement.

Plus spécifiquement, la présente invention concerne un procédé de synthèse de l'ivabradine de formule (I), de ses sels d'addition à un acide pharmaceutiquement acceptable et de ses hydrates,
caractérisé en ce que l'on soumet le composé de formule (V) : 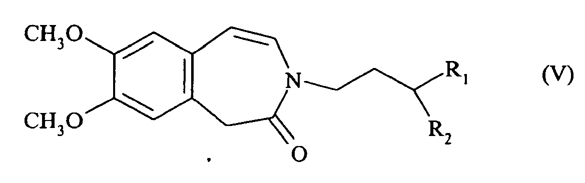 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
à une réaction d'hydrogénation catalytique,
puis que l'on soumet le composé de formule (VI) ainsi obtenu : 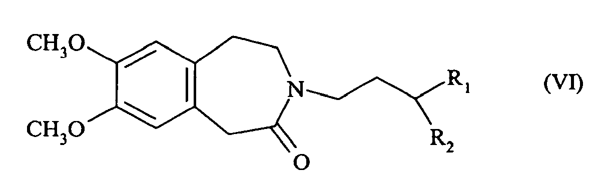 dans laquelle R₁ et R₂ sont tels que définis précédemment,
à une réaction avec le composé de formule (VII) : 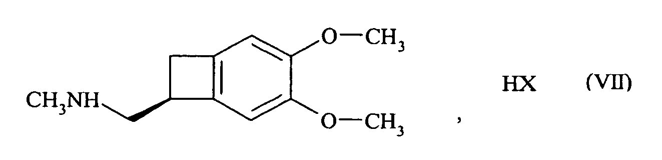 dans laquelle HX représente un acide pharmaceutiquement acceptable,
en présence d'hydrogène et d'un catalyseur,
pour conduire directement, après filtration du catalyseur et isolement, au sel d'addition de l'ivabradine à l'acide HX, que l'on soumet éventuellement, lorsqu'on souhaite accéder à l'ivabradine libre, à l'action d'une base.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, nitrique, citrique, ascorbique, oxalique, méthanesulfonique, benzènesulfonique et camphorique.

Ce procédé permet d'accéder directement aux sels d'addition de l'ivabradine, et en particulier à son chlorhydrate, en une seule étape à partir du sel correspondant de l'amine de formule (II), avec une excellente pureté et un très bon rendement.

Parmi les catalyseurs utilisables pour la réaction d'hydrogénation du composé de formule (V), on peut citer à titre non limitatif le palladium, le platine, le nickel, le ruthénium, le rhodium, ainsi que leurs dérivés, notamment sous forme supportée ou sous forme d'oxydes. Le catalyseur de la réaction d'hydrogénation du composé de formule (V) est préférentiellement le palladium sur charbon.

La température de la réaction d'hydrogénation du composé de formule (V) est préférentiellement comprise entre 20 et 100°C, plus préférentiellement entre 40 et 80°C, encore plus préférentiellement entre 45 et 65°C.

La pression d'hydrogène lors de la réaction d'hydrogénation du composé de formule (V) est préférentiellement comprise entre 1 et 220 bars, plus préférentiellement entre 1 et 100 bars, encore plus préférentiellement entre 1 et 30 bars.

La réaction d'hydrogénation du composé de formule (V) est préférentiellement effectuée dans un solvant non acide.
Parmi les solvants non acides préférés, on citera à titre non limitatif les acétates, les alcools, préférentiellement l'éthanol, le méthanol ou l'isopropanol, le tétrahydrofurane, le toluène, le dichlorométhane et le xylène.

De façon avantageuse, le composé intermédiaire de formule (VI) n'est pas isolé, et le brut réactionnel est engagé tel quel dans la réaction d'amination réductrice.

Parmi les catalyseurs utilisables pour la réaction d'amination réductrice entre le composé de formule (VI) et le composé de formule (VII), on peut citer à titre non limitatif le palladium, le platine, le nickel, le ruthénium, le rhodium, ainsi que leurs dérivés, notamment sous forme supportée ou sous forme d'oxydes.

Le catalyseur de la réaction d'amination réductrice entre le composé de formule (VI) et le composé de formule (VII) est préférentiellement le palladium sur charbon.

La température de la réaction d'amination réductrice entre le composé de formule (VI) et le composé de formule (VII) est préférentiellement comprise entre 30 et 120°C, plus préférentiellement entre 40 et 100°C, encore plus préférentiellement entre 60 et 95°C.

La pression d'hydrogène lors de la réaction d'amination réductrice entre le composé de formule (VI) et le composé de formule (VII) est préférentiellement comprise entre 1 et 220 bars, plus préférentiellement entre 1 et 100 bars, encore plus préférentiellement entre 10 et 60 bars.

Dans le procédé selon l'invention, les composés de formules (V) et (VI) préférentiellement utilisés sont les composés de formules (Va) et (VIa), cas particuliers des composés de formules (V) et (VI) pour lesquels R₁ et R₂ forment ensemble avec l'atome de carbone qui les porte un cycle 1,3-dioxane, 1,3-dioxolane ou 1,3-dioxépane.

Les composés de formule (Va), cas particulier des composés de formules (V) pour lesquels R₁ et R₂ forment ensemble avec l'atome de carbone qui les porte un cycle 1,3-dioxane, 1,3-dioxolane ou 1,3-dioxépane, ainsi que les composés de formule (VI), sont des produits nouveaux, utiles comme intermédiaires de synthèse dans l'industrie chimique ou pharmaceutique, notamment dans la synthèse de l'ivabradine et de ses sels d'addition à un acide pharmaceutiquement acceptable, et font à ce titre partie intégrante de la présente invention.

Le procédé préféré selon l'invention est le procédé utilisant comme intermédiaire de synthèse le composé de formule (VIIa), cas particulier des composés de formule (VII) pour lequel HX représente l'acide chlorhydrique, conduisant ainsi au chlorhydrate de l'ivabradine de formule (Ia).

Dans ce cas, le procédé selon la présente invention conduit au chlorhydrate de l'ivabradine sous une forme cristalline, la forme α, qui est bien définie, parfaitement reproductible et qui présente notamment des caractéristiques intéressantes de filtration, de séchage, de stabilité et de facilité de formulation.

Cette forme cristalline α est nouvelle et constitue un autre aspect de la présente invention.

La forme cristalline α du chlorhydrate de l'ivabradine est caractérisée par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,1 | 1341 | 177 | 0,1338 | 21,486 |
| 2 | 7,7 | 1266 | 146 | 0,117 | 11,440 |
| 3 | 8, | 1325 | 197 | 0,1506 | 10,923 |
| 4 | 10,4 | 1630 | 161 | 0,1004 | 8,488 |
| 5 | 11,8 | 753 | 87 | 0,1171 | 7,473 |
| 6 | 12,1 | 292 | 29 | 0,1004 | 7,301 |
| 7 | 13,2 | 917 | 106 | 0,1171 | 6,709 |
| 8 | 13,8 | 875 | 130 | 0,1506 | 6,423 |
| 9 | 15,3 | 281 | 37 | 0,1338 | 5,790 |
| 10 | 16,2 | 816 | 108 | 0,1338 | 5,478 |
| 11 | 16,5 | 2784 | 459 | 0,1673 | 5,381 |
| 12 | 17,4 | 1308 | 129 | 0,1004 | 5,106 |
| 13 | 18,1 | 455 | 52 | 0,1171 | 4,885 |
| 14 | 19,4 | 223 | 37 | 0,1673 | 4,569 |
| 15 | 20,2 | 3282 | 487 | 0,1506 | 4,389 |
| 16 | 20,6 | 305 | 45 | 0,1506 | 4,310 |
| 17 | 21,3 | 550 | 91 | 0,1673 | 4,165 |
| 18 | 21,9 | 1266 | 230 | 0,184 | 4,050 |
| 19 | 22,4 | 416 | 41 | 0,1004 | 3,972 |
| 20 | 23,0 | 262 | 35 | 0,1338 | 3,861 |
| 21 | 23,3 | 184 | 27 | 0,1506 | 3,814 |
| 22 | 24,4 | 309 | 51 | 0,1673 | 3,651 |
| 23 | 25,0 | 362 | 72 | 0,2007 | 3,566 |
| 24 | 25,7 | 1076 | 142 | 0,1338 | 3,459 |
| 25 | 26,5 | 2925 | 579 | 0,2007 | 3,363 |
| 26 | 26,8 | 821 | 135 | 0,1673 | 3,325 |
| 27 | 27,8 | 488 | 97 | 0,2007 | 3,212 |
| 28 | 28,4 | 620 | 123 | 0,2007 | 3,142 |
| 29 | 29,2 | 428 | 56 | 0,1338 | 3,057 |

L'invention s'étend aussi aux compositions pharmaceutiques renfermant comme principe actif la forme cristalline α du chlorhydrate de l'ivabradine avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention.

Le spectre de diffraction X sur poudre a été mesuré avec les conditions expérimentales suivantes :
- Diffractomètre PANalytical X'Pert Pro, détecteur X'Celerator,
- Tension 45 KV, intensité 40mA,
- Montage *θ*-*θ*,
- Filtre K*β* (Ni),
- fente de soller sur le faisceau incident et sur le faisceau diffracté : 0,04 rad,
- angle fixe des fentes de divergence : 1/8°,
- Masque : 10 mm,
- Fente anti-diffusion : 1/4°,
- Mode de mesure : continu de 3° à 30°, avec une incrémentation de 0,017°,
- Temps de mesure par pas : 19,7 s,
- Temps total : 4 min 32 s,
- Vitesse de mesure : 0,108 °/s,
- Température de mesure : ambiante.

### EXEMPLE 1 : Forme cristalline alpha du chlorhydrate de 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2H-3-benzazépin-2-one

Dans un autoclave, charger 5,5 kg de 3-[2-(1,3-dioxolan-2-yl)éthyl]-7,8-diméthoxy-1,3-dihydro-2*H*-3-benzazépin-2-one, 27,5 1 d'éthanol et 550 g de palladium sur charbon.
Purger à l'azote puis à l'hydrogène, chauffer à 55°C, puis hydrogéner à cette température sous une pression de 5 bars jusqu'à absorption de la quantité théorique d'hydrogène.
Ramener ensuite à température ambiante, puis décompresser l'autoclave.

Ajouter ensuite 4 kg du chlorhydrate de la (7S)-3,4-diméthoxybicyclo[4.2.0]octa-1,3,5-trién-7-yl]-N-méthylméthanamine, 11 l d'éthanol, 5,5 l d'eau et 1 kg de palladium sur charbon.
Purger à l'azote puis à l'hydrogène, chauffer à 85°C, puis hydrogéner à cette température sous une pression de 30 bars jusqu'à absorption de la quantité théorique d'hydrogène.
Revenir ensuite à température ambiante, purger l'autoclave, puis filtrer le mélange réactionnel, distiller les solvants puis isoler le chlorhydrate d'ivabradine par cristallisation dans un mélange toluène/1-méthyl-2-pyrrolidinone.

Le chlorhydrate d'ivabradine est ainsi obtenu avec un rendement de 85 % et une pureté chimique supérieure à 99 %.

### Diagramme de diffraction X sur poudre :

Le profil de diffraction des rayons X de la poudre (angles de diffraction) de la forme α du chlorhydrate de l'ivabradine est donné par les raies significatives rassemblées dans le tableau suivant :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,1 | 1341 | 177 | 0,1338 | 21,486 |
| 2 | 7,7 | 1266 | 146 | 0,117 | 11,440 |
| 3 | 8,1 | 1325 | 197 | 0,1506 | 10,923 |
| 4 | 10,4 | 1630 | 161 | 0,1004 | 8,488 |
| 5 | 11,8 | 753 | 87 | 0,1171 | 7,473 |
| 6 | 12,1 | 292 | 29 | 0,1004 | 7,301 |
| 7 | 13,2 | 917 | 106 | 0,1171 | 6,709 |
| 8 | 13,8 | 875 | 130 | 0,1506 | 6,423 |
| 9 | 15,3 | 281 | 37 | 0,1338 | 5,790 |
| 10 | 16,2 | 816 | 108 | 0,1338 | 5,478 |
| 11 | 16,5 | 2784 | 459 | 0,1673 | 5,381 |
| 12 | 17,4 | 1308 | 129 | 0,1004 | 5,106 |
| 13 | 18,1 | 455 | 52 | 0,1171 | 4,885 |
| 14 | 19,4 | 223 | 37 | 0,1673 | 4,569 |
| 15 | 20,2 | 3282 | 487 | 0,1506 | 4,389 |
| 16 | 20,6 | 305 | 45 | 0,1506 | 4,310 |
| 17 | 21,3 | 550 | 91 | 0,1673 | 4,165 |
| 18 | 21,9 | 1266 | 230 | 0,184 | 4,050 |
| 19 | 22,4 | 416 | 41 | 0,1004 | 3,972 |
| 20 | 23,0 | 262 | 35 | 0,1338 | 3,861 |
| 21 | 23,3 | 184 | 27 | 0,1506 | 3,814 |
| 22 | 24,4 | 309 | 51 | 0,1673 | 3,651 |
| 23 | 25,0 | 362 | 72 | 0,2007 | 3,566 |
| 24 | 25,7 | 1076 | 142 | 0,1338 | 3,459 |
| 25 | 26,5 | 2925 | 579 | 0,2007 | 3,363 |
| 26 | 26,8 | 82 | 135 | 0,1673 | 3,325 |
| 27 | 27,8 | 488 | 97 | 0,2007 | 3,212 |
| 28 | 28,4 | 620 | 123 | 0,2007 | 3,142 |
| 29 | 29,2 | 428 | 56 | 0,1338 | 3,057 |

### EXEMPLE 2 : Composition pharmaceutique

Formule de préparation pour 1000 comprimés dosés à 5 mg d'ivabradine base:

| | |
|---|---|
| Composé de l'exemple 1 | 5,39 g |
| Amidon de maïs | 20 g |
| Silice colloïdale anhydre | 0,2 g |
| Mannitol | 63,91 g |
| PVP | 10 g |
| Stéarate de magnésium | 0,5 g |

## Revendications

1. Procédé de synthèse de l'ivabradine de formule (I) : 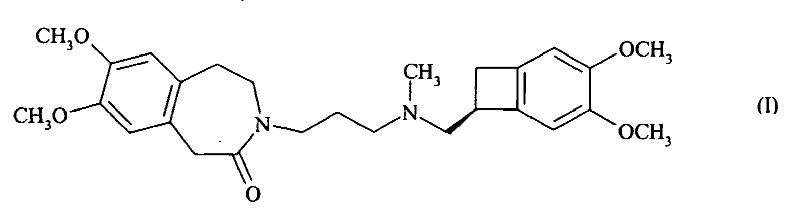 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de ses hydrates,
**caractérisé en ce que** l'on soumet le composé de formule (V) : 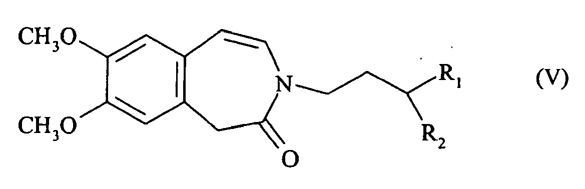 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
à une réaction d'hydrogénation catalytique,
puis que l'on soumet le composé de formule (VI) ainsi obtenu : 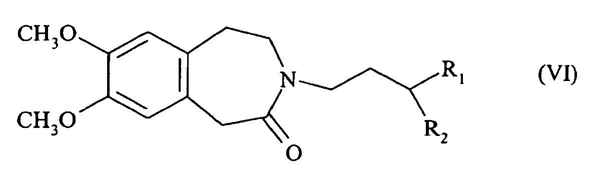 dans laquelle R₁ et R₂ sont tels que définis précédemment,
à une réaction avec le composé de formule (VII) : 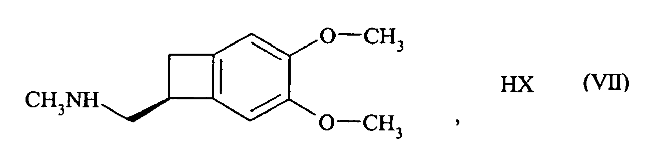 dans laquelle HX représente un acide pharmaceutiquement acceptable,
en présence d'hydrogène et d'un catalyseur,
pour conduire directement, après filtration du catalyseur et isolement, au sel d'addition de l'ivabradine à l'acide HX, que l'on soumet éventuellement, lorsqu'on souhaite accéder à l'ivabradine libre, à l'action d'une base.

2. Procédé de synthèse selon la revendication 1, dans lequel le catalyseur de la réaction d'hydrogénation du composé de formule (V) est le palladium sur charbon.

3. Procédé de synthèse selon l'une quelconque des revendications 1 ou 2, dans lequel la pression d'hydrogène lors de la réaction d'hydrogénation du composé de formule (V) est comprise entre 1 et 220 bars.

4. Procédé de synthèse selon l'une quelconque des revendications 1 à 3, dans lequel la température de la réaction d'hydrogénation du composé de formule (V) est comprise entre 20 et 100°C.

5. Procédé de synthèse selon la revendication 4, dans lequel la température de la réaction d'hydrogénation du composé de formule (V) est comprise entre 40 et 80°C.

6. Procédé de synthèse selon l'une quelconque des revendications 1 à 5, dans lequel le composé intermédiaire de formule (VI) n'est pas isolé.

7. Procédé de synthèse selon l'une quelconque des revendications 1 à 6, dans lequel le catalyseur de la réaction entre le composé de formule (VI) et le composé de formule (VII) est le palladium sur charbon.

8. Procédé de synthèse selon l'une quelconque des revendications 1 à 7, dans lequel la pression d'hydrogène lors de la réaction entre le composé de formule (VI) et le composé de formule (VII) est comprise entre 1 et 220 bars.

9. Procédé de synthèse selon l'une quelconque des revendications 1 à 8, dans lequel la température de la réaction entre les composés de formule (VI) et (VII) est comprise entre 30 et 120°C.

10. Procédé de synthèse selon la revendication 9, dans lequel la température de la réaction entre les composés de formule (VI) et (VII) est comprise entre 40 et 100°C.

11. Procédé de synthèse selon l'une quelconque des revendications 1 à 10, **caractérisé en ce qu'**il utilise les composés de formules (Va) et (VIa), cas particuliers des composés de formules (V) et (VI) pour lesquels R₁ et R₂ forment ensemble avec l'atome de carbone qui les porte un cycle 1,3-dioxane, 1,3-dioxolane ou 1,3-dioxépane.

12. Procédé de synthèse selon l'une quelconque des revendications 1 à 11, **caractérisé en ce qu'**il utilise l'intermédiaire de formule (VIIa), cas particulier des composés de formule (VII) pour lequel HX représente l'acide chlorhydrique, conduisant ainsi au chlorhydrate de l'ivabradine.

13. Composé de formule (Va) : 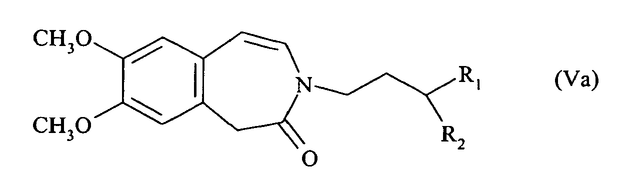 dans laquelle R₁ et R₂ forment ensemble avec l'atome de carbone qui les porte un cycle 1,3-dioxane, 1,3-dioxolane ou 1,3-dioxépane.

14. Composé de formule (VI) : 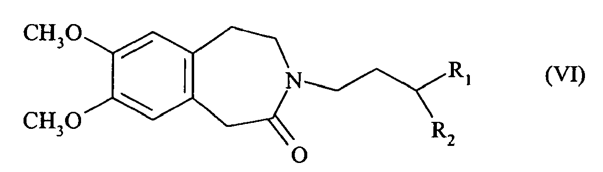 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane.

15. Forme cristalline α du chlorhydrate de l'ivabradine de formule (Ia) : 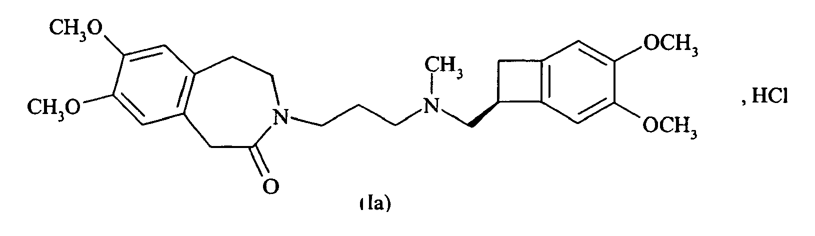 **caractérisée par** le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :
| **Raie n°** | **Angle 2 thêta (degrés)** | **Hauteur (coups)** | **Surface (coups x degrés)** | **FWHM (degrés)** | **Distance inter-réticulaire** (Å) |
|---|---|---|---|---|---|
| 1 | 4,1 | 1341 | 177 | 0,1338 | 21,486 |
| 2 | 7,7 | 1266 | 146 | 0,1171 | 11,440 |
| 3 | 8,1 | 1325 | 197 | 0,1506 | 10,923 |
| 4 | 10,4 | 1630 | 161 | 0,1004 | 8,488 |
| 5 | 11,8 | 753 | 87 | 0,1171 | 7,473 |
| 6 | 12,1 | 292 | 29 | 0,1004 | 7,301 |
| 7 | 13,2 | 917 | 106 | 0,1171 | 6,709 |
| 8 | 13,8 | 875 | 130 | 0,1506 | 6,423 |
| 9 | 15,3 | 281 | 37 | 0,1338 | 5,790 |
| 10 | 16,2 | 816 | 108 | 0,1338 | 5,478 |
| 11 | 16,5 | 2784 | 459 | 0,1673 | 5,381 |
| 12 | 17,4 | 1308 | 129 | 0,1004 | 5,106 |
| 13 | 18,1 | 455 | 52 | 0,1171 | 4,885 |
| 14 | 19,4 | 223 | 37 | 0,1673 | 4,569 |
| 15 | 20,2 | 3282 | 487 | 0,1506 | 4,389 |
| 16 | 20,6 | 305 | 45 | 0,1506 | 4,310 |
| 17 | 21,3 | 550 | 91 | 0,1673 | 4,165 |
| 18 | 21,9 | 1266 | 230 | 0,184 | 4,050 |
| 19 | 22,4 | 416 | 41 | 0,1004 | 3,972 |
| 20 | 23,0 | 262 | 35 | 0,1338 | 3,861 |
| 21 | 23,3 | 184 | 27 | 0,1506 | 3,814 |
| 22 | 24,4 | 309 | 51 | 0,1673 | 3,651 |
| 23 | 25,0 | 362 | 72 | 0,2007 | 3,566 |
| 24 | 25,7 | 1076 | 142 | 0,1338 | 3,459 |
| 25 | 26,5 | 2925 | 579 | 0,2007 | 3,363 |
| 26 | 26,8 | 821 | 135 | 0,1673 | 3,325 |
| 27 | 27,8 | 488 | 97 | 0,2007 | 3,212 |
| 28 | 28,4 | 620 | 123 | 0,2007 | 3,142 |
| 29 | 29,2 | 428 | 56 | 0,1338 | 3,057 |

16. Composition pharmaceutique contenant comme principe actif la forme cristalline α du chlorhydrate de l'ivabradine selon la revendication 15, en combinaison avec un ou plusieurs véhicules inertes, non toxiques et pharmaceutiquement acceptables.

17. Utilisation de la forme cristalline α du chlorhydrate de l'ivabradine selon la revendication 15, pour la fabrication de médicaments utiles comme bradycardisants.

18. Utilisation de la forme cristalline α du chlorhydrate de l'ivabradine selon la revendication 15, pour la fabrication de médicaments utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

## Claims

1. Process for the synthesis of ivabradine of formula (I): 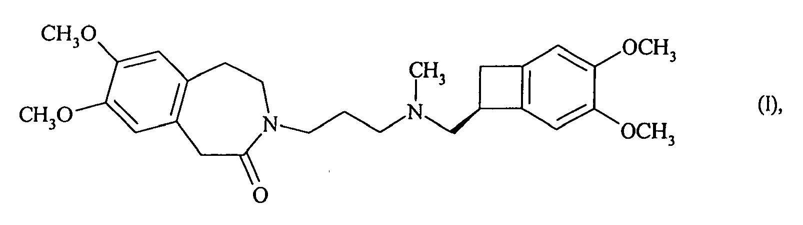 or 3-{3-[{[(7S)-3,4-dimethoxybicyclo[4.2.0]octa-1,3,5-trien-7-yl]methyl}(methyl)amino]-propyl}-7,8-dimethoxy-1,3,4,5-tetrahydro-2*H*-3-benzazepin-2-one,
addition salts thereof with a pharmaceutically acceptable acid, and hydrates thereof,
**characterised in that** the compound of formula (V) : 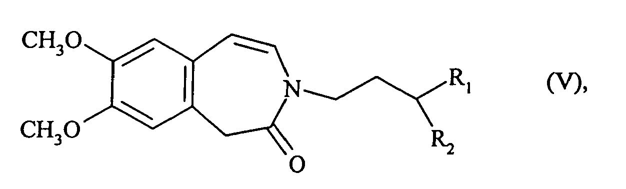 wherein R₁ and R₂, which may be the same or different, each represent a linear or branched (C₁-C₈)alkoxy group or form, together with the carbon atom carrying them, a 1,3-dioxane, 1,3-dioxolane or 1,3-dioxepane ring,
is subjected to a catalytic hydrogenation reaction,
and then the compound of formula (VI) thereby obtained : 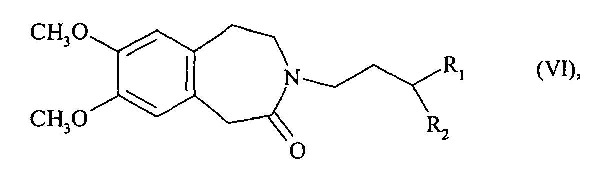 wherein R₁ and R₂ are as defined hereinbefore,
is subjected to a reaction with the compound of formula (VII) : 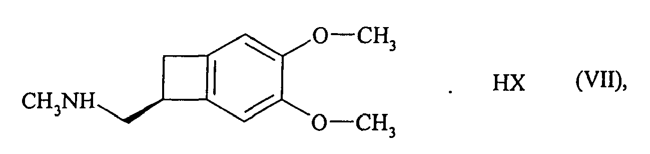 wherein HX represents a pharmaceutically acceptable acid,
in the presence of hydrogen and a catalyst,
to yield directly, after filtering off the catalyst and isolation, the addition salt of ivabradine with the acid HX, which is optionally subjected, when it desired to obtain free ivabradine, to the action of a base.

2. Synthesis process according to claim 1, wherein the catalyst for the hydrogenation reaction of the compound of formula (V) is palladium-on-carbon.

3. Synthesis process according to either claim 1 or claim 2, wherein the hydrogen pressure during the hydrogenation reaction of the compound of formula (V) is from 1 to 220 bars.

4. Synthesis process according to any one of claims 1 to 3, wherein the temperature of the hydrogenation reaction of the compound of formula (V) is from 20 to 100°C.

5. Synthesis process according to claim 4, wherein the temperature of the hydrogenation reaction of the compound of formula (V) is from 40 to 80°C.

6. Synthesis process according to any one of claims 1 to 5, wherein the intermediate compound of formula (VI) is not isolated.

7. Synthesis process according to any one of claims 1 to 6, wherein the catalyst for the reaction between the compound of formula (VI) and the compound of formula (VII) is palladium-on-carbon.

8. Synthesis process according to any one of claims 1 to 7, wherein the hydrogen pressure during the reaction between the compound of formula (VI) and the compound of formula (VII) is from 1 to 220 bars.

9. Synthesis process according to any one of claims 1 to 8, wherein the temperature of the reaction between the compounds of formulae (VI) and (VII) is from 30 to 120°C.

10. Synthesis process according to claim 9, wherein the temperature of the reaction between the compounds of formulae (VI) and (VII) is from 40 to 100°C.

11. Synthesis process according to any one of claims 1 to 10, **characterised in that** it uses compounds of formulae (Va) and (VIa), particular cases of the compounds of formulae (V) and (VI) wherein R₁ and R₂ form, together with the carbon atom carrying them, a 1,3-dioxane, 1,3-dioxolane or 1,3-dioxepane ring.

12. Synthesis process according to any one of claims 1 to 11, **characterised in that** it uses the intermediate of formula (VIIa), a particular case of the compounds of formula (VII) wherein HX represents hydrochloric acid, thereby yielding ivabradine hydrochloride.

13. Compound of formula (Va) : 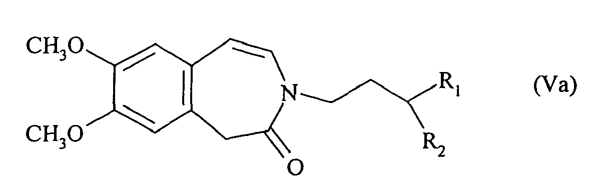 wherein R₁ and R₂ form, together with the carbon atom carrying them, a 1,3-dioxane, 1,3-dioxolane or 1,3-dioxepane ring.

14. Compound of formula (VI) : 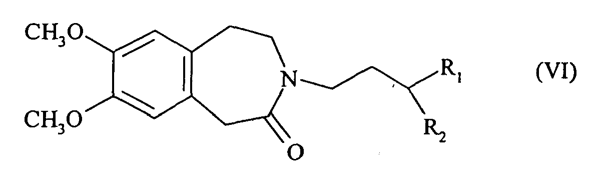 wherein R₁ and R₂, which may be the same or different, each represent a linear or branched (C₁-C₈)alkoxy group or form, together with the carbon atom carrying them, a 1,3-dioxane, 1,3-dioxolane or 1,3-dioxepane ring.

15. α crystalline form of ivabradine hydrochloride of formula (Ia): 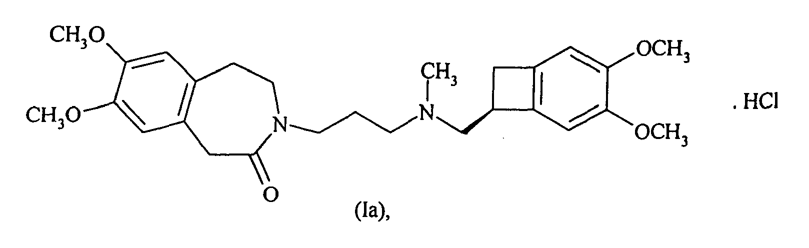 **characterised by** the following powder X-ray diffraction diagram measured using a PANalytical X'Pert Pro diffractometer together with an X' Celerator detector and expressed in terms of line position (Bragg's angle 2 theta, expressed in degrees), line height (expressed in counts), line area (expressed in counts x degrees), line width at half-height ("FWHM", expressed in degrees) and interplanar distance d (expressed in Å) :
| **Line no.** | **Angle 2 theta (degrees)** | **Height (counts)** | **Area (counts x degrees)** | **FWHM (degrees)** | **Interplanar distance (Å)** |
|---|---|---|---|---|---|
| 1 | 4.1 | 1341 | 177 | 0.1338 | 21.486 |
| 2 | 7.7 | 1266 | 146 | 0.1171 | 11.440 |
| 3 | 8.1 | 1325 | 197 | 0.1506 | 10.923 |
| 4 | 10.4 | 1630 | 161 | 0.1004 | 8.488 |
| 5 | 11.8 | 753 | 87 | 0.1171 | 7.473 |
| 6 | 12.1 | 292 | 29 | 0.1004 | 7.301 |
| 7 | 13.2 | 917 | 106 | 0.1171 | 6.709 |
| 8 | 13.8 | 875 | 130 | 0.1506 | 6.423 |
| 9 | 15.3 | 281 | 37 | 0.1338 | 5.790 |
| 10 | 16.2 | 816 | 108 | 0.1338 | 5.478 |
| 11 | 16.5 | 2784 | 459 | 0.1673 | 5.381 |
| 12 | 17.4 | 1308 | 129 | 0.1004 | 5.106 |
| 13 | 18.1 | 455 | 52 | 0.1171 | 4.885 |
| 14 | 19.4 | 223 | 37 | 0.1673 | 4.569 |
| 15 | 20.2 | 3282 | 487 | 0.1506 | 4.389 |
| 16 | 20.6 | 305 | 45 | 0.1506 | 4.310 |
| 17 | 21.3 | 550 | 91 | 0.1673 | 4.165 |
| 18 | 21.9 | 1266 | 230 | 0.184 | 4.050 |
| 19 | 22.4 | 416 | 41 | 0.1004 | 3.972 |
| 20 | 23.0 | 262 | 35 | 0.1338 | 3.861 |
| 21 | 23.3 | 184 | 27 | 0.1506 | 3.814 |
| 22 | 24.4 | 309 | 51 | 0.1673 | 3.651 |
| 23 | 25.0 | 362 | 72 | 0.2007 | 3.566 |
| 24 | 25.7 | 1076 | 142 | 0.1338 | 3.459 |
| 25 | 26.5 | 2925 | 579 | 0.2007 | 3.363 |
| 26 | 26.8 | 821 | 135 | 0.1673 | 3.325 |
| 27 | 27.8 | 488 | 97 | 0.2007 | 3.212 |
| 28 | 28.4 | 620 | 123 | 0.2007 | 3.142 |
| 29 | 29.2 | 428 | 56 | 0.1338 | 3.057 |

16. Pharmaceutical composition comprising as active ingredient the α crystalline form of ivabradine hydrochloride according to claim 15, in combination with one or more pharmaceutically acceptable, inert and non-toxic carriers.

17. Use of the α crystalline form of ivabradine hydrochloride according to claim 15 in the manufacture of medicaments which are of use as bradycardics.

18. Use of the α crystalline form of ivabradine hydrochloride according to claim 15 in the manufacture of medicaments which are of use in the treatment or prevention of various clinical situations of myocardial ischaemia such as angina pectoris, myocardial infarct and associated rhythm disturbances, and also in various pathologies involving rhythm disturbances, especially supraventricular rhythm disturbances, and in heart failure.

## Patentansprüche

1. Verfahren zur Synthese von Ivabradin der Formel (I): 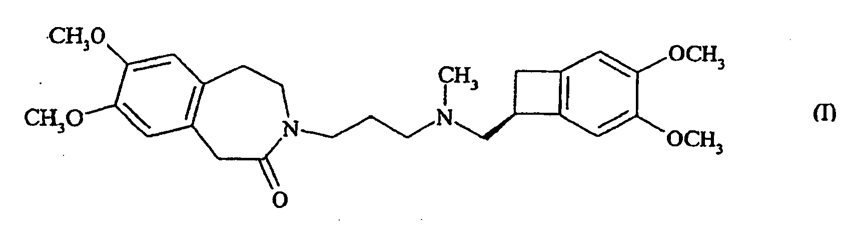 oder von 3-{3-[{[(7S)-3,4-Dimethoxybicyclo[4.2.0]octa-1.3.5-trien-7-yl]-methyl}(methyl)-amino]-propyl}-7,8-dimethoxy-1,3,4,5-tetrahydro-2*H*-3-benzazepin-2-on, von seinen Additionssalzen mit einer pharmazeutisch annehmbaren Säure und seinen Hydraten,
**dadurch gekennzeichnet, daß** man die Verbindung der Formel (V): 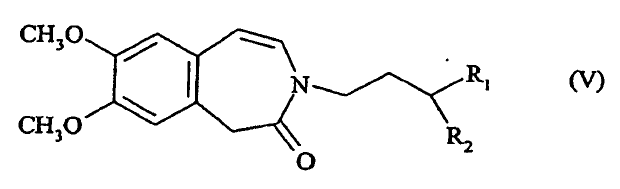 in der R₁ und R₂, die gleichartig oder verschieden sind, jeweils eine geradkettige oder verzweigte (C₁-C₈)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden,
einer katalytischen Hydrierungsreaktion unterwirft
und dann die in dieser Weise erhaltene Verbindung der Formel (VI): 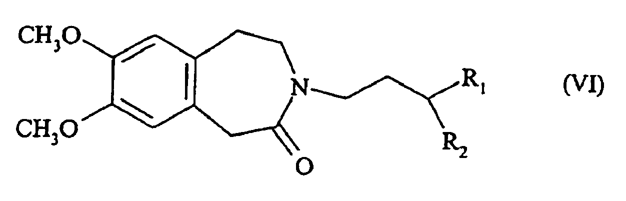 in der R₁ und R₂ die oben angegebenen Bedeutungen besitzen,
in Gegenwart von Wasserstoff und eines Katalysators mit der Verbindung der Formel (VII): 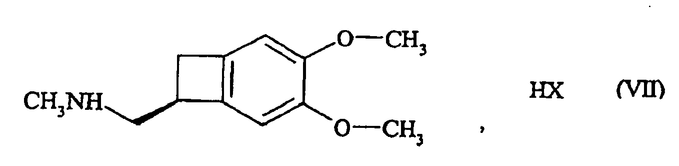 in der HX eine pharmazeutisch annehmbare Säure bedeutet, umsetzt, so daß man direkt, nach dem Abfiltrieren des Katalysators und der Isolierung das Additionssalz von Ivabradin mit der Säure HX erhält, welches man, wenn man freies Ivabradin erhalten will, der Einwirkung einer Base unterwirft.

2. Syntheseverfahren nach Anspruch 1, worin der Katalysator für die Hydrierreaktion der Verbindung der Formel (V) Palladium-auf-Kohlenstoff ist.

3. Syntheseverfahren nach einem der Ansprüche 1 oder 2, worin der Wasserstoffdruck bei der Hydrierreaktion der Verbindung der Formel (V) zwischen 1 und 220 bar liegt.

4. Syntheseverfahren nach einem der Ansprüche 1 bis 3, worin die Temperatur der Hydrierungsreaktion der Verbindung der Formel (V) zwischen 20 und 100°C liegt.

5. Syntheseverfahren nach Anspruch 4, worin die Temperatur der Hydrierungsreaktion der Verbindung der Formel (V) zwischen 40 und 80°C liegt.

6. Syntheseverfahren nach einem der Ansprüche 1 bis 5, worin die Zwischenverbindung der Formel (VI) nicht isoliert wird.

7. Syntheseverfahren nach einem der Ansprüche 1 bis 6, worin der Katalysator für die Umsetzung der Verbindung der Formel (VI) mit der Verbindung der Formel (VII) Palladium-auf-Kohlenstoff ist.

8. Syntheseverfahren nach einem der Ansprüche 1 bis 7, worin der Wasserstoffdruck bei der Umsetzung der Verbindung der Formel (VI) mit der Verbindung der Formel (VII) zwischen 1 und 220 bar liegt.

9. Syntheseverfahren nach einem der Ansprüche 1 bis 8, worin die Temperatur bei der Umsetzung der Verbindungen der Formel (VI) und (VII) zwischen 30 und 120°C liegt.

10. Syntheseverfahren nach Anspruch 9, worin die Temperatur der Umsetzung der Verbindungen (VI) und (VII) zwischen 40 und 100°C liegt.

11. Syntheseverfahren nach einem der Ansprüche 1 bis 10, **dadurch gekennzeichnet, daß** man die Verbindungen der Formeln (Va) und (VIa) verwendet, Sonderfällen der Verbindungen der Formeln (V) und (VI), worin R₁ und R₂ gemeinsam mit dem sie tragenden Kohlenstoffatom einen 1,3-Dioxan-, 1,3-Dioxolan- oder 1,3-Dioxepan-Ring bilden.

12. Syntheseverfahren nach einem der Ansprüche 1 bis 11, **dadurch gekennzeichnet, daß** man das Zwischenprodukt der Formel (VIIa) verwendet, ein Sonderfall der Verbindungen der Formel (VII), worin HX Chlorwasserstoffsäure bedeutet, und man dieser Weise das Hydroxchlorid von Ivabradin erhält.

13. Verbindung der Formel (Va): 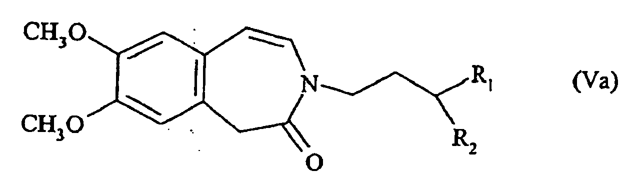 in der R₁ und R₂ gemeinsam mit dem sie tragenden Kohlenstoffatom einen 1,3-Dioxan-, 1,3-Dioxolan oder 1,3-Dioxepan-Ring bilden.

14. Verbindung der Formel (VI): 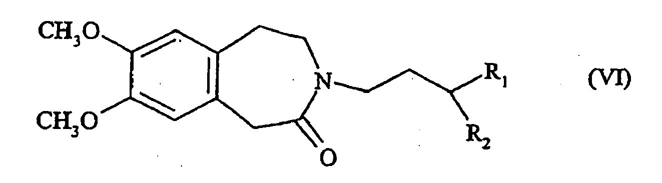 in der R₁ und R₂, die gleichartig oder verschieden sind, jeweils eine geradkettige oder verzweigte (C₁-C₈)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden.

15. α-Kristallform von Ivabradin-Hydrochlorid der Formel (Ia): 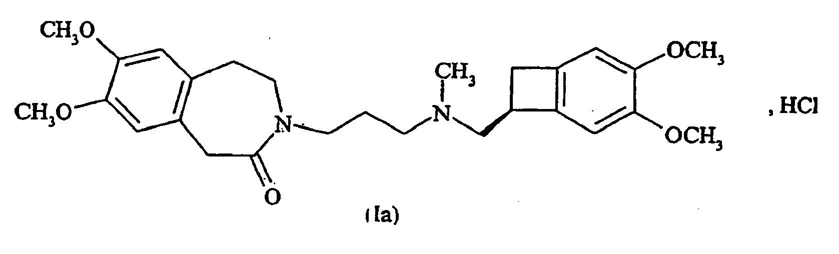 **gekennzeichnet durch** das nachfolgende Pulver-Röntgenbeugungsdiagramm, welches mit einem PANalytical X'Pert Pro-Diffraktometer, der mit einem X'Celerator-Detektor ausgerüstet ist, gemessen worden ist und ausgedrückt ist in Hinblick auf die Linienposition (Bragg-Winkel 2 Theta, angegebenen in Grad), die Linienhöhe (angegeben in Zählimpulsen), die Linienfläche (ausgedrückt in Zählimpulsen x Grad), und Linienbreite bei halber Höhe ("FWHM", angegebenen in Grad) und den Netzebenenabstand d (angegeben in Å):
| **Linie Nr.** | **Winkel 2 Theta (Grad)** | **Höhe (Zahlimpulse)** | **Oberfläche (Zahlimpulse x Grad)** | **FWHM (Grad)** | **Netzebenenabstand (Å)** |
|---|---|---|---|---|---|
| 1 | 4,1 | 1341 | 177 | 0,1338 | 21,486 |
| 2 | 7,7 | 1266 | 146 | 0,117 | 11,440 |
| 3 | 8,1 | 1325 | 197 | 0,1506 | 10,923 |
| 4 | 10,4 | 1630 | 16 | 0,1004 | 8,488 |
| 5 | 11,8 | 753 | 87 | 0,117 | 7,473 |
| 6 | 12, | 292 | 29 | 0,1004 | 7,301 |
| 7 | 13,2 | 917 | 106 | 0,1171 | 6,709 |
| 8 | 13,8 | 875 | 130 | 0,1506 | 6,423 |
| 9 | 15,3 | 281 | 37 | 0,1338 | 5,790 |
| 10 | 16,2 | 816 | 108 | 0,1338 | 5,478 |
| 11 | 16,5 | 2784 | 459 | 0,1673 | 5,381 |
| 12 | 17,4 | 1308 | 129 | 0,1004 | 5,106 |
| 13 | 18,1 | 455 | 52 | 0,1171 | 4,885 |
| 14 | 19,4 | 223 | 37 | 0,1673 | 4,569 |
| 15 | 20,2 | 3282 | 487 | 0,1506 | 4,389 |
| 16 | 20,6 | 305 | 45 | 0,1506 | 4,310 |
| 17 | 21,3 | 550 | 91 | 0,1673 | 4,165 |
| 18 | 21,9 | 1266 | 230 | 0,184 | 4,050 |
| 19 | 22,4 | 416 | 41 | 0,1004 | 3,972 |
| 20 | 23,0 | 262 | 35 | 0,1338 | 3,861 |
| 21 | 23,3 | 184 | 27 | 0,1506 | 3,814 |
| 22 | 24,4 | 309 | 51 | 0,1673 | 3,651 |
| 23 | 25,0 | 362 | | 72 0,2007 | 3,566 |
| 24 | 25,7 | 1076 | 142 | 0,1338 | 3,459 |
| 25 | 26,5 | 2925 | 579 | 0,2007 | 3,363 |
| 26 | 26,8 | 821 | 135 | 0,1673 | 3,325 |
| 27 | 27,8 | 488 | 97 | 0,2007 | 3,212 |
| 28 | 28,4 | 620 | 123 | 0,2007 | 3,142 |
| 29 | 29,2 | 428 | 56 | 0,1338 | 3,057 |

16. Pharmazeutische Zubereitung enthaltend als Wirkstoff die α-Kristallform von Ivabardin-Hydrochlorid gemäß Anspruch 15 in Kombination mit einem oder mehreren inerten, nicht-toxischen und pharmazeutisch annehmbaren Hilfsstoffen.

17. Verwendung der α-Kristallform von Ivabradin-Hydrochlorid nach Anspruch 15 für die Herstellung von Arzneimitteln, die als bradykardisierende Mittel nützlich sind.

18. Verwendung der α-Kristallform von Ivabradin-Hydrochlorid nach Anspruch 15 für die Herstellung von Arzneimitteln, die nützlich sind bei der Behandlung oder der Vorbeugung von verschiedenartigen klinischen Situationen der Myokardischämie, wie Angina pectoris, Myokardinfarkt und damit verknüpften Rhythmusstörungen, sowie für die Behandlung verschiedenartiger pathologischer Zustände, welche Rhythmusstörungen umfassen, insbesondere supra-ventikulären Rhythmusstörungen und Herzinsuffizienz.
